# DEMANDE DE BREVET EUROPEEN

(11) **EP 1 669 072 A1**
(43) Date de publication de la demande: **14.06.2006**
(21) Numéro de dépôt: 05292625.0
(22) Date de dépôt: 09.12.2005
(51) Int. Cl.: A61K 31/5415, A61K 31/542

(54) **Dérivés de benzothiazine et benzothiadiazine, leur procédé de préparation et les compositions pharmaceutiques qui les contiennent**

(30) Priorité: 10.12.2004 FR 0413170
(71) Demandeur: Les Laboratoires Servier, 92415 Courbevoie Cedex (FR)
(72) Inventeur: Desos, Patrice, 92270 Bois-Colombes (FR); Cordi, Alexis, 92150 Suresnes (FR); Lestage, Pierre, 78170 La Celle-St-Cloud (FR); Danober, Laurence, 78360 Montesson (FR)

(57) **Abrégé**

Composés de formule (I) : 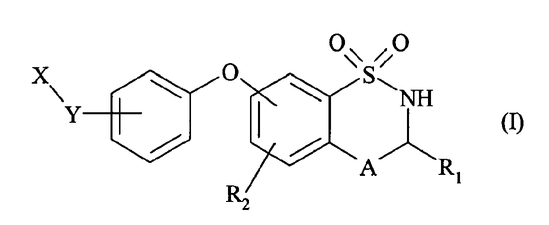 dans laquelle :
- R₁: représente hydrogène, alkyle ou cycloalkyle,
- R₂: représente hydrogène, halogène ou hydroxy,
- A: représente CR₃R₄ ou NR₃, où R₃ et R₄ sont tels que définis dans la description,
- Y: représente une chaîne alkylène telle que décrite dans la description,
- X: représente NR₅R₆, S(O)ₙR₇, OR₈, C(O)R₉, amidino ou hétérocycle,
leurs isomères ainsi que leurs sels d'addition.

Médicaments.

## Description

La présente invention concerne de nouveaux dérivés de benzothiazine et benzothiadiazine, leur procédé de préparation et les compositions pharmaceutiques qui les contiennent.

Il est désormais reconnu que les aminoacides excitateurs, et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545).

Par ailleurs, d'innombrables travaux ont démontré, durant les dernières années, l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits très récemment comme modulant positivement les récepteurs AMPA des cellules neuronales (Journal of Neurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Enfin, le brevet EP 692 484 décrit un dérivé de benzothiadiazine possédant une activité facilitatrice sur le courant AMPA et la demande de brevet WO 99/42456 décrit, entre autres, certains dérivés de benzothiadiazine en tant que modulateurs des récepteurs AMPA.

Les dérivés de benzothiazine et de benzothiadiazine, objets de la présente invention, outre le fait qu'ils soient nouveaux, présentent, de manière surprenante, des activités pharmacologiques sur le courant AMPA nettement supérieures à celles des composés de structures proches décrits dans l'Art Antérieur. Ils sont utiles, en tant que modulateurs AMPA, pour le traitement ou la prévention des désordres mnémocognitifs associés à l'âge, aux syndromes anxieux ou dépressifs, aux maladies neurodégénératives progressives, à la maladie d'Alzheimer, à la maladie de Pick, à la chorée d'Huntington, à la schizophrénie, aux séquelles des maladies neurodégénératives aiguës, aux séquelles de l'ischémie et aux séquelles de l'épilepsie.

Plus spécifiquement, la présente invention concerne les composés de formule (I) : 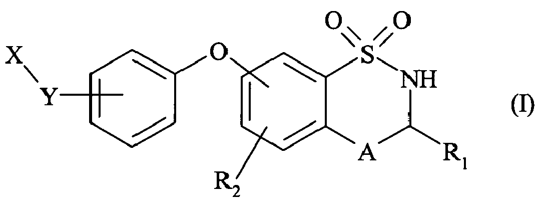 dans laquelle :
- R₁: représente un atome d'hydrogène, un groupement alkyle (C₁-C₆) linéaire ou ramifié ou un groupement cycloalkyle (C₃-C₇),
- R₂: représente un atome d'hydrogène, d'halogène ou un groupement hydroxy,
- A: représente un groupement CR₃R₄ ou un groupement NR₃, dans lesquels
R₃ représente un atome d'hydrogène ou un groupement alkyle (C₁-C₆) linéaire ou ramifié,
et R₄ représente un atome d'hydrogène ou d'halogène, ou bien
- A: représente un atome d'azote et forme avec le groupement -CHR₁- adjacent le cycle 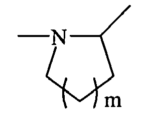 dans lequel m représente 1, 2 ou 3,
- Y: représente une chaîne alkylène (C₂-C₆) éventuellement substituée par un ou plusieurs groupements identiques ou différents alkyle (C₁-C₆) linéaire ou ramifié ou atomes d'halogène, et dont un des groupements -CH₂- peut être remplacé par un groupement 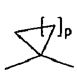 dans lequel p vaut 1, 2, 3 ou 4,
ou Y représente un groupement 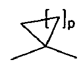 tel que défini précédemment,
- X: représente un groupement NR₅R₆, S(O)ₙR₇, OR₈, C(O)R₉, amidino (éventuellement substitué par un ou deux groupements identiques ou différents choisis parmi alkyle(C₁-C₆) linéaire ou ramifié, hydroxy, alkoxy (C₁-C₆) linéaire ou ramifié, et 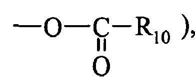 ou un groupement hétérocyclique dans lesquels :
R₅ représente un atome d'hydrogène, un groupement alkyle (C₁-C₆) linéaire ou ramifié, S(O)ₜR₁₁, COR₁₂ ou P(O)OR₁₃OR₁₄,
R₆ représente un atome d'hydrogène, ou un groupement alkyle (C₁-C₆) linéaire ou ramifié, ou bien R₅ et R₆ forment ensemble avec l'atome d'azote qui les porte un groupement hétérocyclique,
R₈ représente un groupement alkyle (C₁-C₆) linéaire ou ramifié ou un groupement C(O)R₁₅,
R₉ représente un groupement hydroxy, alkoxy (C₁-C₆) linéaire ou ramifié ou amino (éventuellement substitué par un ou deux groupements, identiques ou différents, alkyle (C₁-C₆) linéaire ou ramifié), R₇, R₁₀, R₁₁, R₁₂, R₁₃, R₁₄ et R₁₅, identiques ou différents, représentent chacun un atome d'hydrogène ; un groupement alkyle (C₁-C₆) linéaire ou ramifié éventuellement substitué par un ou plusieurs atomes d'halogène ; un groupement arylalkyle (C₁-C₆) linéaire ou ramifié ; ou un groupement aryle,
n et t, identiques ou différents, représentent 0, 1 ou 2,
leurs énantiomères et diastéréoisomères ainsi que leurs sels d'addition à un acide ou à une base pharmaceutiquement acceptable,
étant entendu que :
◆ par groupement hétérocyclique, on comprend groupement aromatique ou non, monocyclique ou bicyclique, contenant un à quatre hétéroatomes, identiques ou différents, choisis parmi azote, oxygène et soufre, éventuellement substitué par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, alkoxycarbonyle (C₁-C₆) linéaire ou ramifié, oxo, thioxo, carboxy, acyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié), et alkyl (C₁-C₆) sulfonylamino,
◆ par groupement aryle, on comprend groupement aromatique monocyclique ou groupement bicyclique dans lequel au moins un des cycles est aromatique, contenant 5 à 10 atomes de carbone, éventuellement substitué par un ou plusieurs groupements, identiques ou différents, choisis parmi halogène, alkyle (C₁-C₆) linéaire ou ramifié (éventuellement substitué par un ou plusieurs groupements hydroxy), alkoxy (C₁-C₆) linéaire ou ramifié, polyhalogénoalkyle (C₁-C₆) linéaire ou ramifié, alkoxycarbonyle (C₁-C₆) linéaire ou ramifié, oxo, thioxo, alkylthio (C₁-C₆) linéaire ou ramifié, carboxy, acyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié ou acyle (C₁-C₆) linéaire ou ramifié), aminocarbonyle (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié), mono ou di(alkyl(C₁-C₆)sulfonyl)amino, mono ou di(trifluorométhylsulfonyl) amino, PO(ORₐ)(OR_{b}) (dans lequel Rₐ, R_{b}, identiques ou différents, représentent un atome d'hydrogène ou un groupement alkyle (C₁-C₆) linéaire ou ramifié), benzyloxy, et phényle (éventuellement substitué par un ou plusieurs groupements, identiques ou différents, choisis parmi halogène, alkyle (C₁-C₆) linéaire ou ramifié, polyhalogénoalkyle (C₁-C₆) linéaire ou ramifié, hydroxy et alkoxy (C₁-C₆) linéaire ou ramifié).

Parmi les acides pharmaceutiquement acceptables, on peut citer, à titre non limitatif, les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer, à titre non limitatif, l'hydroxyde de sodium, l'hydroxyde de potassium, la triéthylamine, la tertbutylamine, etc...

Plus particulièrement, l'invention concerne les composés de formule (I) pour lesquels A représente un atome d'azote et forme avec le groupement -CHR₁- adjacent le cycle 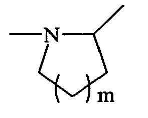 dans lequel m représente 1, 2 ou 3, et plus particulièrement le cycle

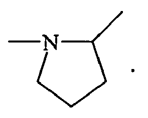

Le groupement R₂ préféré est l'atome d'hydrogène.

Y représente préférentiellement une chaîne alkylène contenant 2 ou 3 atomes de carbone, non substituée ou substituée. Les substitutions éventuelles préférées de la chaîne alkylène représentant Y sont l'atome de fluor et le groupement méthyle.

Les groupements X préférés sont les groupements NR₅R₆ ou C(O)R₉, et plus particulièrement le groupement NHSO₂R₁₁ où R₁₁ représente préférentiellement un groupement alkyle (C₁-C₆) linéaire ou ramifié.

Encore plus particulièrement, l'invention concerne les composés de formule (I) qui sont :
- le *N*-(2-{4-[(5,5-dioxido-2,3,3a,4-tétrahydro-1*H*-pyrrolo[2,1-*c*][1,2,4]benzothiadiazin-7-yl) oxy]phényl}éthyl)méthanesulfonamide,
- l'acide 3-{4-[(5,5-dioxido-2,3,3a,4-tétrahydro-1*H*-pyrrolo[2,1-*c*][1,2,4]benzothiadiazin-yl) oxy]phényl}propanoïque,
- le 3-{4-[(5,5-dioxido-2,3,3a,4-tétrahydro-1*H*-pyrrolo[2,1-*c*][1,2,4]benzothiadiazin-7-yl)oxy] phényl}propanamide,
- le 3-{4-[(5,5-dioxido-2,3,3a,4-tétrahydro-1*H*-pyrrolo[2,1-*c*][1,2,4]benzothiadiazin-7-yl) oxy]phényl}-*N*,*N*-diméthylpropanamide,
- le *N*-(3-{4-[(5,5-dioxido-2,3,3a,4-tétrahydro-1*H*-pyrrolo[2,1-*c*][1,2,4]benzothiadiazin-7-yl) oxy]phényl}propyl)méthanesulfonamide,
- le *N*-(2-{4-[(5,5-dioxido-2,3,3a,4-tétrahydro-1*H*-pyrrolo[2,1-*c*][1,2,4]benzothiadiazin-7-yl) oxy]phényl}-2-fluoropropyl)-2-propanesulfonamide,
- le *N*-(2-{4-[(5,5-dioxido-2,3,3a,4-tétrahydro-1*H*-pyrrolo[2,1-*c*][1,2,4]benzothiadiazin-7-yl) oxy]phényl}-2-fluoropropyl)méthanesulfonamide,
- le *N*-(2-{4-[(5,5-dioxido-2,3,3a,4-tétrahydro-1*H*-pyrrolo[2,1-*c*][1,2,4]benzothiadiazin-7-yl) oxy]phényl}propyl)méthanesulfonamide,
- le *N*-(2-{4-[(5,5-dioxido-2,3,3a,4-tétrahydro-1*H*-pyrrolo[2,1-*c*][1,2,4]benzothiadiazin-7-yl) oxy]phényl}éthyl)-*N*,*N*-diméthylamine,
- le *N*-(1-{4-[(5,5-dioxido-2,3,3a,4-tétrahydro-1*H*-pyrrolo[2,1-*c*][1,2,4]benzothiadiazin-7-yl) oxy]phényl}cyclopropyl)méthanesulfonamide.

L'invention concerne également le procédé de préparation des composés de formule (I) caractérisé en ce que l'on utilise comme produit de départ le composé de formule (II) : 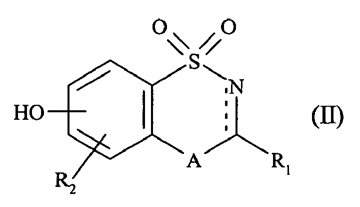 dans laquelle A, R₁ et R₂ sont tels que définis dans la formule (I) et la représentation ----- signifie que la liaison est simple ou double,
que l'on fait réagir, en présence d'acétate de cuivre, avec un dérivé d'acide boronique de formule (III) : 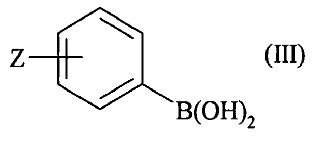 dans laquelle Z représente un groupement acyle (C₁-C₆) linéaire ou ramifié, un groupement -Y-X dans lequel X et Y sont tels que définis dans la formule (I) ou un groupement -Y-X' dans lequel Y est tel que défini dans la formule (I) et X' représente un groupement cyano ou carboxy,
pour conduire directement, ou après réduction (lorsque la représentation ----- représente une double liaison) par un hydrure métallique et/ou par transformation éventuelle du groupement X' ou du groupement acyle, au composé de formule (I) : 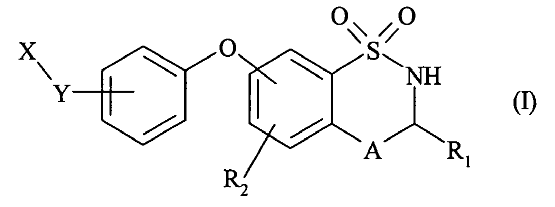 composé de formule (I) que l'on purifie, le cas échéant, selon une technique classique de purification, dont on sépare éventuellement les isomères, selon une technique classique de séparation, et que l'on transforme, si on le souhaite, en ses sels d'addition, à un acide ou à une base pharmaceutiquement acceptable.

Les composés de formule (II), tels que définis précédemment, sont obtenus par des réactions classiques de la chimie organique et plus particulièrement par le procédé décrit dans WO03053979.

L'invention s'étend aussi aux compositions pharmaceutiques renfermant comme principe actif un composé de formule (I) avec un ou plusieurs excipients inertes, non toxiques et appropriés.
Parmi les compositions pharmaceutiques, selon l'invention, on pourra citer plus particulièrement celles qui conviennent pour l'administration orale, parentérale (intraveineuse ou sous-cutanées), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention mais ne la limitent en aucune façon.

Les produits de départ utilisés sont des produits connus ou préparés selon des modes opératoires connus.
Les structures des composés, décrits dans les exemple, ont été déterminées selon les techniques spectrophotométriques usuelles (infrarouge, RMN, spectrométrie de masse...).

### Exemple 1 : N-(2-{4-[(5,5-Dioxido-2,3,3a,4-tétrahydro-1H-pyrrolo[2,1-c][1,2,4]benzo thiadiazin-7-yl)oxy]phényl}éthyl)méthanesulfonamide

Dans un erlen de 100 ml sont introduits 25 ml de CH₂Cl₂, 240 µl (2,96 mmol) de pyridine, 238 mg (0,99 mmol) de 2,3,3a,4-tétrahydro-1*H*-pyrrolo[2,1-*c*][1,2,4]benzothiadiazin-7-ol 5,5-dioxide, 2,5 g de tamis moléculaire 4 Å, 340 mg (1,48 mmol) d'acide 4-{2-[(méthylsulfonyl)amino]éthyl}phénylboronique et 270 mg (1,48 mmol) de Cu(OAc)₂. La suspension est agitée vigoureusement à température ambiante en laissant l'erlen ouvert à l'air libre. Après 4 h 30 la réaction est diluée dans 50 ml de CH₂Cl₂ supplémentaires et la suspension est filtrée. Le filtrat est évaporé à sec et le résidu est purifié par 2 chromatographies successives sur colonne de silice : la première chromatographie est éluée par CH₂Cl₂/Acétone (96/4), la deuxième par CH₂Cl₂/AcOEt (70/30). L'huile incolore obtenue est cristallisée par trituration dans un mélange Et₂O et quelques gouttes d'isopropanol pour conduire après filtration au composé du titre sous forme d'une poudre blanche.
*Point de fusion* : 99-102°C

**Microanalyse élémentaire :**

| | *C* | *H* | *N* | *S* |
|---|---|---|---|---|
| *% théorique* | 52,16 | 5,30 | 9,60 | 14,66 |
| *% expérimental* | 52,57 | 5,42 | 9,67 | 14,92 |

### Exemple 2 : Acide 3-{4-[(5,5-dioxido-2,3,3a,4-tétrahydro-1H-pyrrolo[2,1-c][1,2,4]benzothiadiazin-7-yl)oxy]phényl}propanoïque

### Stade A : 3-{4-[(5,5-Dioxido-2,3-dihydro-1H-pyrrolo[2,1-c][1,2,4]benzothiadiazin-7-yl) oxy]phényl}propanoate de méthyle

Le produit du titre est obtenu par réaction du 2,3-dihydro-1*H*-pyrrolo[2,1-*c*][1,2,4]benzo-thiadiazin-7-ol 5,5-dioxide avec l'acide 4-(3-méthoxy-3-oxopropyl)phényl boronique selon le mode opératoire de l'Exemple 1 mais le temps de réaction est porté à 48 heures. La purification est réalisée par chromatograhie sur colonne de silice en éluant par un mélange CH₂Cl₂/acétone 95/5.
*Point de fusion* : 138-140°C

**Microanalyse élémentaire :**

| | *C* | *H* | *N* | *S* |
|---|---|---|---|---|
| *% théorique* | 59,99 | 5,03 | 7,00 | 8,01 |
| *% expérimental* | 59,96 | 5,12 | 6,90 | 7,51 |

### Stade B : Acide 3-{4-[(5,5-dioxido-2,3-dihydro-1H-pyrrolo[2,1-c][1,2,4]benzothiadiazin-7-yl)oxy]phényl}propanoïque

Une suspension de 580 mg (1,45 mmol) du produit obtenu au stade A est agitée à reflux dans 9 ml d'HCl 1N pendant 5 heures. Le milieu réactionnel est ensuite dilué dans l'eau et la suspension est filtrée pour conduire au produit du titre sous la forme d'un solide blanc.
*Point de fusion :* 216-222°C

**Microanalyse élémentaire :**

| | *C* | *H* | *N* | *S* |
|---|---|---|---|---|
| *% théorique* | 59,06 | 4,70 | 7,25 | 8,30 |
| *% expérimental* | 59,34 | 4,87 | 7,27 | 8,35 |

### Stade C : Acide 3-{4-[(5,5-dioxido-2,3,3a,4-tétrahydro-1H-pyrrolo[2,1-c][1,2,4] benzothiadiazin-7-yl)oxy]phényl}propanoïque

A une suspension de 210 mg (0,54 mmol) du produit du stade précédent dans 6 ml d'éthanol sont rajoutés 62 mg (1,63 mmol) de NaBH₄. Après 2 heures d'agitation à température ambiante la solution réactionnelle est refroidie dans un bain de glace et acidifiée par addition de HCl 1N. La suspension gommeuse est extraite par CH₂Cl₂, la phase organique est ensuite lavée par une solution aqueuse saturée en NaCl, séchée sur MgSO₄ et évaporée. Le résidu est trituré dans Et₂O et le solide blanc formé est filtré pour conduire au produit du titre.
*Point de fusion* : 178-183°C

**Microanalyse élémentaire :**

| | *C* | *H* | *N* | *S* |
|---|---|---|---|---|
| *% théorique* | 58,75 | 5,19 | 7,21 | 8,25 |
| *% expérimental* | 58,73 | 5,24 | 7,11 | 8,41 |

### Exemple 3 : 3-{4-[(5,5-Dioxido-2,3,3a,4-tétrahydro-1H-pyrrolo[2,1-c][1,2,4]benzothiadiazin-7-yl)oxy]phényl}propanamide

### Stade A : 3-{4-[(5,5-Dioxido-2,3-dihydro-1H-pyrrolo[2,1-c][1,2,4]benzothiadiazin-7-yl) oxy]phényl}propanamide

A une suspension (500 mg, 1,30 mmol) du produit obtenu au stade B de l'exemple 2 dans 25 ml de CH₂Cl₂ sont ajoutés 415 mg (1,30 mmol) de TBTU. La suspension est agitée 10 min à température ambiante et 293 µl (1,68 mmol) de diisopropyléthylamine sont ajoutées. Le milieu réactionnel passe en solution et l'agitation est poursuivie 10 min. La solution est ensuite saturée en ammoniac gaz et l'agitation est poursuivie 30 min. Une chromatographie sur couche mince (AcOEt) indique une disparition totale du produit de départ. Le milieu réactionnel est alors acidifié par addition d'HCl 1N, la phase organique est décantée, lavée (eau puis NaCl saturé), séchée (MgSO₄) et évaporée. Le résidu est trituré dans Et₂O et le solide blanc formé est filtré pour conduire au produit du titre.
*Point de fusion* : 178-183°C

### Stade B : 3-{4-[(5,5-Dioxido-2,3,3a,4-tétrahydro-1H-pyrrolo[2,1-c][1,2,4] benzothiadiazin-7-yl)oxy]phényl}propanamide

On procède selon le mode opératoire du stade C de l'Exemple 2, à partir du composé obtenu au stade A.
*Point de fusion* : 195-198°C

**Microanalyse élémentaire :**

| | *C* | *H* | *N* | *S* |
|---|---|---|---|---|
| *% théorique* | 58,9 | 5,46 | 10,85 | 8,28 |
| *% expérimental* | 58,55 | 5,47 | 10,40 | 8,24 |

### Exemple 4 : 3-{4-[(5,5-Dioxido-2,3,3a,4-tétrahydro-1H-pyrrolo[2,1-c][1,2,4] benzothiadiazin-7-yl)oxy]phényl}-N,N-diméthylpropanamide

### Stade A : 3-{4-[(5,5-Dioxido-2,3-dihydro-1H-pyrrolo[2,1-c][1,2,4]benzothiadiazin-7-yl)oxy]phéyl}-N,N-diméthylpropanamide

On procède comme dans le stade A de l'Exemple 3 mais en remplaçant l'ammoniac gaz par la diméthylamine 2M dans le THF. La purification est réalisée par chromatograhie sur colonne de silice en éluant par un mélange CH₂Cl₂/MeOH 98/2.
*Point de fusion :* 77-80°C

### Stade B : 3-{4-[(5,5-Dioxido-2,3,3a,4-tétrahydro-1H-pyrrolo[2,1-c][1,2,4] benzothiadiazin-7-yl)oxy]phényl}-N,N-diméthylpropanamide

On procède selon le mode opératoire du stade C de l'Exemple 2 à partir du composé obtenu au stade A. La purification est réalisée par chromatograhie sur colonne de silice en éluant par un mélange CH₂Cl₂/acétone 90/10.
*Point de fusion* : 189-192°C

**Microanalyse élémentaire :**

| | *C* | *H* | *N* | *S* |
|---|---|---|---|---|
| *% théorique* | 60,70 | 6,06 | 10,11 | 7,72 |
| *% expérimental* | 61,24 | 6,37 | 10,16 | 7,68 |

### Exemple 5 : N-(3-{4-[(5,5-Dioxido-2,3,3a,4-tétrahydro-1H-pyrrolo [2,1-c][1,2,4] benzothiadiazin-7-yl)oxy]phényl}propyl)méthanesulfonamide

### Stade A : (3-{4-[(5,5-Dioxido-2,3,3a,4-tétrahydro-1H-pyrrolo[2,1-c][1,2,4] benzothiadiazin-7-yl)oxy]phényl}propyl)amine

A une suspension (390 mg, 1,01 mmol) du produit obtenu au stade A de l'Exemple 3 dans 20 ml de THF sont ajoutés par petites portions 192 mg (5,06 mmol) de LiAlH₄ et la réaction est agitée à reflux pendant 1 h 30. Le milieu réactionnel est refroidi dans un bain de glace et est traité par addition goutte à goutte d'une solution aqueuse de NH₄Cl. Les sels d'alumines sont filtrés, rincés par du THF puis de l'acétone et le filtrat est concentré. Celui-ci est dilué dans CH₂Cl₂ et l'amine du titre est extraite par HCl 1N. La phase aqueuse acide est lavée par CH₂Cl₂, alcalinisée avec NaHCO₃ 10% et extraite 3 fois par AcOEt. Les phases organiques sont rassemblées, séchées sur MgSO₄ et évaporées. Le résidu est trituré dans Et₂O et le solide blanc formé est filtré pour conduire au produit du titre.
*Point de fusion* : 126-131°C

### Stade B : N-(3-{4-[(5,5-Dioxido-2,3,3a,4-tétrahydro-1 H-pyrrolo [2,1-c][1,2,4] benzothiadiazin-7-yl)oxy]phényl}propyl)méthanesulfonamide

A une solution à 0°C du produit obtenu au stade A (106 mg, 0,28 mmol) dans 2 ml de CH₂Cl₂ refroidies dans un bain de glace sont ajoutés 59 µL (0,43 mmol) de triéthylamine puis goutte à goutte 74 mg (0,43 mmol) d'anhydride méthanesulfonique en solution dans 2 ml de CH₂Cl₂. La solution réactionnelle est agitée 2 heures en laissant la température remonter à l'ambiante. La phase organique est lavée par HCl 1N puis par une solution aqueuse saturée en NaCl et est séchée sur MgSO₄. Après évaporation le composé du titre est purifié par chromatographie sur colonne de silice éluée par un mélange CH₂Cl₂/acétone 95/5.
*Point de fusion :* 65-69°C

**Microanalyse élémentaire :**

| | *C* | *H* | *N* | *S* |
|---|---|---|---|---|
| *% théorique* | 53,20 | 5,58 | 9,31 | 14,20 |
| *% expérimental* | 53,24 | 5,64 | 9,12 | 14,40 |

### Exemple 6 : N-((2S)-2-{4-[(5,5-Dioxido-2,3,3a,4-tétrahydro-1H-pyrrolo [2,1-c][1,2,4] benzothiadiazin-7-yl)oxy]phényl}-2-fluoropropyl)propane-2-sulfonamide

Le produit du titre est obtenu par réaction du 2,3,3a,4-tétrahydro-1*H*-pyrrolo[2,1-*c*][1,2,4] benzothiadiazin-7-ol 5,5-dioxide avec l'acide 4-{1-fluoro-2-[(isopropylsulfonyl)amino]-1-méthyléthyl}phényl boronique selon le mode opératoire de l'Exemple 1 mais le temps de réaction est portée à 16 heures. La purification est réalisée par 2 chromatograhies successives sur colonne de silice en éluant pour la première avec un mélange CH₂Cl₂/MeOH 98/2 et pour la deuxième avec un mélange cyclohexane/acétate d'éthyle 70/30.
*Point de fusion :* 90°C

**Microanalyse élémentaire :**

| | *C* | *H* | *N* | *S* |
|---|---|---|---|---|
| *% théorique* | 53,10 | 5,67 | 8,44 | 12,89 |
| *% expérimental* | 53,81 | 5,76 | 8,21 | 12,72 |

### Exemple 7 : N-(2-{4-[(5,5-Dioxido-2,3,3a,4-tétrahydro-1H-pyrrolo[2,1-c][1,2,4] benzothiadiazin-7-yl)oxy]phényl}-2-fluoropropyl)méthanesulfonamide

### Stade A : 1-{4-[(5,5-Dioxido-2,3,3a,4-tétrahydro-1H-pyrrolo[2,1-c][1,2,4] benzothiadiazin-7-yl)oxy]phényl}éthanone

Le produit du titre est obtenu par réaction du 2,3,3a,4-tétrahydro-1*H*-pyrrolo[2,1-*c*] [1,2,4]benzothiadiazin-7-ol 5,5-dioxide avec l'acide 4-acétylphényl boronique selon le mode opératoire de l'Exemple 1 mais le temps de réaction est porté à 16 heures. La purification est réalisée par chromatograhie sur colonne de silice en éluant avec un mélange CH₂Cl₂/acétone 98/2.
*Point de fusion* : 150-152°C

### Stade B : 1-Amino-2-{4-[(5,5-dioxido-2,3,3a,4-tétrahydro-1H-pyrrolo[2,1-c][1,2,4] benzothiadiazin-7-yl)oxy]phényl}propan-2-ol

A une solution contenant 1,0 g (2,79 mmol) du produit obtenu au stade A, 23 mg (0,09 mmol) d'éther couronne 18-crown-6, 18,5 mg (0,28 mmol) de KCN dans 25 ml de THF, sont rajoutés goutte à goutte 840 µl (6,26 mmol) de TMSCN. La solution réactionnelle est agitée 3 heures à température ambiante puis 424 mg (11,2 mmol) de LiAlH₄ sont ajoutés par petites portions. Après 1 h 30, l'excès d'hydrure est hydrolysé par addition goutte à goutte d'une solution aqueuse saturée en NaCl. Le milieu réactionnel est filtré, le solide rincé plusieurs fois avec du THF et le filtrat est évaporé pour donner une meringue blanche correspondant au produit du titre.
*Point de fusion :* 76-78°C

### Stade C : N-(2-{4-[(5,5-Dioxido-2,3,3a,4-tétrahydro-1H-pyrrolo[2,1-c][1,2,4] benzothiadiazin-7-yl)oxy]phényl}-2-hydroxypropyl)méthanesulfonamide

A une solution du produit obtenu au stade B (640 mg, 1,64 mmol) dans 25 ml de CH₂Cl₂ refroidies dans un bain de glace sont ajoutés 354 µL (2,53 mmol) de triéthylamine puis goutte à goutte 441 mg (2,53 mmol) d'anhydride méthanesulfonique en solution dans 10 ml de CH₂Cl₂. La solution réactionnelle est agitée 2 heures en laissant la température remonter à l'ambiante. La phase organique est lavée par HCl 1N puis NaCl saturé et est séchée sur MgSO₄. Après évaporation le résidu est purifié par chromatographie sur colonne de silice éluée par un mélange CH₂Cl₂/MeOH 96/4.
*Point de fusion :* 90-92°C

### Stade D : N-(2-{4-[(5,5-Dioxido-2,3,3a,4-tétrahydro-1H-pyrrolo[2,1-c][1,2,4] benzothiadiazin-7-yl)oxy]phényl}-2-fluoropropyl)méthanesulfonamide

A une solution du produit obtenu au stade C (230 mg, 0,49 mmol) dans 15 ml de CH₂Cl₂ refroidie dans un bain de glace sont ajoutés goutte à goutte 133 µL (0,99 mmol) de DAST. La solution réactionnelle est agitée 2 heures en laissant la température remonter à l'ambiante. La phase organique est lavée par H₂O puis NaCl saturé et est séchée sur MgSO₄. Après évaporation, le résidu est purifié par chromatographie sur colonne de silice éluée par un mélange CH₂Cl₂/MeOH 99/1.
*Point de fusion* : 84-86°C

**Microanalyse élémentaire :**

| | *C* | *H* | *N* | *S* |
|---|---|---|---|---|
| *% théorique* | 51,16 | 5,15 | 8,95 | 13,66 |
| *% expérimental* | 51,34 | 5,48 | 8,91 | 14,16 |

### Exemple 8 : N-(2-{4-[(5,5-Dioxido-2,3,3a,4-tétrahydro-1H-pyrrolo [2,1-c][1,2,4] benzothiadiazin-7-yl)oxy]phényl}propyl)méthanesulfonamide

### Stade A : 2-{4-[(5,5-Dioxido-2,3,3a,4-tétrahydro-1H-pyrrolo[2,1-c][1,2,4] benzothiadiazin-7-yl)oxy]phényl}propanenitrile

A une solution du produit du stade A de l'exemple 7 (700 mg, 1,95 mmol) dans 25 ml de 1,2-diméthoxyéthane sont ajoutés 840 mg (4,30 mmol) de tosylméthylisonitrile. La solution réactionnelle est refroidie à -25 °C et 6 ml (6 mmol) de *t*BuOK 1M dans le THF sont ajoutés goutte à goutte. La réaction est agitée 30 min à -25 °c puis est laissée revenir à température ambiante pendant 1 heure. Après addition d'HCl 1N la réaction est extraite par AcOEt, les phases organiques sont rassemblées, lavées par NaCl saturé, séchées (MgSO₄). Après évaporation, le résidu est purifié par chromatographie sur colonne de silice éluée par un mélange CH₂Cl₂/MeOH 99/1 pour conduire à une meringue blanche correspondant au produit du titre.

### Stade B : (2-{4-[(5,5-Dioxido-2,3,3a,4-tétrahydro-1H-pyrrolo[2,1-c][1,2,4] benzothiadiazin-7-yl)oxy]phényl}propyl)amine

A une solution contenant 485 mg (1,31 mmol) du produit du stade A précédent dans 15 ml de THF, sont ajoutés par petites portions 200 mg (5,25 mmol) de LiAlH₄. Après 3 heures de réaction, l'excès d'hydrure est hydrolysé par addition goutte à goutte d'une solution aqueuse saturée en NaCl. Le milieu réactionnel est filtré, le solide rincé plusieurs fois avec du THF et le filtrat est évaporé pour donner une meringue blanche correspondant au produit du titre.

### Stade C : N-(2-{4-[(5,5-Dioxido-2,3,3a,4-tétrahydro-1H-pyrrolo [2,1-c][1,2,4] benzothiadiazin-7-yl)oxy]phényl}propyl)méthanesulfonamide

A une solution du composé obtenu au stade B (394 mg, 1.06 mmol) dans 20 ml de CH₂Cl₂ refroidie dans un bain de glace, sont ajoutés 441 µL (3,17 mmol) de triéthylamine puis goutte à goutte 404 mg (2,32 mmol) d'anhydride méthanesulfonique en solution dans 5 ml de CH₂Cl₂. La solution réactionnelle est agitée 2 heures en laissant la température remonter à l'ambiante. La phase organique est lavée par HCl 1N puis NaCl saturé et est séchée sur MgSO₄. Après évaporation le résidu est purifié par chromatographie sur colonne de silice éluée par un mélange CH₂Cl₂/MeOH 99/1.
*Point de fusion :* 83-85°C

**Microanalyse élémentaire :**

| | *C* | *H* | *N* | *S* |
|---|---|---|---|---|
| *% théorique* | 53,2 | 5,58 | 9,31 | 14,2 |
| *% expérimental* | 53,53 | 5,67 | 9,05 | 14,26 |

### Exemple 9 : (2-{4-[(5,5-Dioxido-2,3,3a,4-tétrahydro-1H-pyrrolo[2,1-c][1,2,4] benzothiadiazin-7-yl)oxy]phényl}éthyl)diméthylamine

### Stade A : {4-[(5,5-Dioxido-2,3,3a,4-tétrahydro-1H-pyrrolo[2,1-c][1,2,4] benzothiadiazin-7-yl)oxy]phényl}acétate de méthyle

Le composé du titre est obtenu par réaction du 2,3,3a,4-tétrahydro-1*H*-pyrrolo[2,1-*c*][1,2,4]benzothiadiazin-7-ol 5,5-dioxide avec l'acide 4-(2-méthoxy-2-oxoéthyl)phényl boronique selon le mode opératoire de l'Exemple 1 mais le temps de réaction est porté à 16 heures. La purification est réalisée par chromatograhie sur colonne de silice en éluant avec un mélange CH₂Cl₂/MeOH 98/2.
*Point de fusion* : 140-142°C

### Stade B : Acide {4-[(5,5-dioxido-2,3,3a,4-tétrahydro-1H-pyrrolo[2,1-c][1,2,4] benzothiadiazin-7-yl)oxy]phényl}acétique

Une suspension de l'ester obtenu au stade A (538 mg, 1,38 mmol) dans 6 ml de NaOH est agitée à 100°C pendant 3 h 30. La solution réactionnelle est acidifiée par HCl 1N et le précipité blanc est filtré pour conduire au produit du titre.
*Point de fusion* : 166-169°C

### Stade C : 2-{4-[(5,5-Dioxido-2,3,3a,4-tétrahydro-1H-pyrrolo[2,1-c][1,2,4] benzothiadiazin-7-yl)oxy]phényl}-N,N-diméthylacétamide

On procède comme dans le stade A de l'Exemple 3 mais en remplaçant l'ammoniac gaz par la diméthylamine 2M dans le THF et l'acide 3-{4-[5,5-dioxo-2,3-dihydro-1*H*-pyrrolo[2,1-c][1,2,4]benzothiadiazin-7-yl)oxy]phenyl}propanoïque par le composé obtenu au stade B. La purification est réalisée par chromatograhie sur colonne de silice en éluant par un mélange CH₂Cl₂/MeOH 99/1 suivie d'une cristallisation dans l'eau.
*Point de fusion* : 154°C

**Microanalyse élémentaire :**

| | *C* | *H* | *N* | *S* |
|---|---|---|---|---|
| *% théorique* | 59,83 | 5,77 | 10,47 | 7,99 |
| *% expérimental* | 59,78 | 5,81 | 10,28 | 7,95 |

### Stade D : (2-{4-[(5,5-Dioxido-2,3,3a,4-tétrahydro-1H-pyrrolo[2,1-c][1,2,4] benzothiadiazin-7-yl)oxy]phényl}éthyl)diméthylamine

On procède comme dans le stade B de l'exemple 8. La purification est réalisée par chromatograhie sur colonne de silice en éluant par un mélange CH₂Cl₂/MeOH/NH₄OH 97/3/0.3 suivie d'une cristallisation dans Et₂O.
*Point de fusion :* 159°C

**Microanalyse élémentaire :**

| | *C* | *H* | *N* | *S* |
|---|---|---|---|---|
| *% théorique* | 61,99 | 6,5 | 10,84 | 8,58 |
| *% expérimental* | 62,25 | 6,64 | 10,50 | 8,25 |

### EXEMPLE 10 : N-(1-{4-[(5,5-Dioxido-2,3,3a,4-tétrahydro-1H-pyrrolo[2,1-c][1,2,4] benzothiadiazin-7-yl)oxy]phényl}cyclopropyl) méthanesulfonamide

### Stade A : [1-(4-Iodophényl)cyclopropyl]amine

A une solution de 4-iodobenzonitrile (5,0 g, 21,83 mmol) dans 140 ml d'Et₂O anhydre sont ajoutés 7,09 ml (24,01 mmol) de Ti(O*i*Pr)₄ et le mélange réactionnel est refroidi à - 65°C. A cette solution sont ensuite ajoutés goutte à goutte 16 ml (48,00 mmol) d'une solution 3M de bromure d'éthyle magnésien dans Et₂O et la réaction est ramenée à température ambiante. L'agitation est poursuivie 1 heure à température ambiante et 5,53 ml (43,66 mmol) de BF₃.OEt₂. sont ajoutés. L'agitation est poursuivie 1 h 15 à température ambiante et 65 ml de HCl 1N sont ajoutés. On obtient une suspension biphasique, ajoute 300 ml d'Et₂O puis 200 ml de NaOH 1N. La suspension est filtrée et le filtrat est extrait 2 fois avec Et₂O. Les phases organiques sont rassemblées, lavées (eau, NaCl saturé), séchées sur MgSO₄ et concentrées à moitié. La solution résiduelle est extraite avec HCl 1N et l'amine attendue est précipitée par alcalinisation avec NaOH concentré.
*Point de fusion :* 69-72°C

**Micro-analyse élémentaire :**

| | *C* | *H* | *N* | *S* |
|---|---|---|---|---|
| *% théorique* | 41,72 | 3,89 | 5,41 | 48,98 |
| *% expérimental* | 41,79 | 3,96 | 5,35 | 49,35 |

### Stade B : N-[1-(4-Iodophényl)cyclopropyl]méthanesulfonamide

A une solution du produit obtenu au stade A (3 g, 11,58 mmol) dans 50 ml de CH₂Cl₂ sont ajoutés 2,7 ml (18,05 mmol) de DBU puis goutte à goutte une solution de 2,09 g (12 mmol) d'anhydride méthane sulfonique diluée dans 20 ml de CH₂Cl₂. La solution est agitée une nuit à température ambiante puis 20 ml d'HCl 1N sont ajoutés. La phase organique est décantée, lavée (NaCl saturé), séchée (MgSO₄) et après évaporation sous vide le résidu est chromatographié sur silice (CH₂Cl₂/AcOEt 95/5) pour conduire au produit du titre.
*Point de fusion :* 101-102°C

### Stade C : N-{1-[4-(4,4,5,5-Tétraméthyl-1,3,2-dioxaborolan-2-yl)phényl]cyclopropyl} méthanesulfonamide

Un mélange de 1,75 g (5,19 mmol) du produit obtenu au stade B, 1,71 g (7,27 mmol) de bispinacolodiboron, 1,53 g (15,57 mmol) d'AcOK, 127 mg (0,156 mmol) de PdCl₂(dppf).CH₂Cl₂ dans 20 ml de DMSO est agité 30 min à 85°C sous azote. A température ambiante sont ajoutés 50 ml d'eau et la phase aqueuse est extraite 3 fois avec AcOEt. Les phases organiques sont rassemblées, lavées (NaCl saturé), séchées (MgSO₄). Après évaporation sous vide le résidu est chromatographié sur silice (CH₂Cl₂/AcOEt 96/4) pour conduire au produit du titre.

**Micro-analyse élémentaire :**

| | *C* | *H* | *N* | *S* |
|---|---|---|---|---|
| *% théorique* | 56,98 | 7,17 | 4,15 | 9,51 |
| *% expérimental* | 57,11 | 7,20 | 4,19 | 9,70 |

### Stade D : Acide (4-{1-[(méthylsulfonyl)amino]cyclopropyl}phényl)boronique

Une suspension de 810 mg (2,40 mmol) du produit obtenu au stade C, 1,95 g (9,12 mmol) de NaIO₄ dans un mélange de 25 ml d'acétone et 7 ml d'une solution aqueuse 1M d'acétate d'ammonium est agitée 24 heures à température ambiante. Le solide est filtré et rincé abondamment avec de l'acétone. Le filtrat est concentré de moitié et la solution résiduelle est extraite avec AcOEt. La phase organique est lavée (NaCl saturé), séchée (MgSO₄) pour conduire, après évaporation sous vide au composé du titre.

### Stade E : N-(1-{4-[(5,5-Dioxido-2,3,3a,4-tétrahydro-1H-pyrrolo[2,1-c][1,2,4] benzothiadiazin-7-yl)oxy]phényl}cyclopropyl)méthanesulfonamide

Une suspension composée de 290 mg (1,21 mmol) de 2,3,3a,4-tétrahydro-1*H*-pyrrolo[2,1-*c*][1,2,4]benzothiadiazin-7-ol 5,5-dioxide, 465 mg (1,82 mmol) du produit obtenu au stade D, 330 mg (1,82 mmol) d'acétate de cuivre (II), 294 µl (3,64 mmol) de pyridine et d'environ 3,5 g de tamis moléculaire 4 Å dans 35 ml de CH₂Cl₂ est agitée pendant 5 heures à température ambiante. Le milieu réactionnel est filtré, rincé avec CH₂Cl₂/MeOH (1/1). Le filtrat est concentré puis directement déposé sur une colonne de silice qui est éluée avec un mélange 96/4 CH₂Cl₂/acétone. Les fractions contenant le produit attendu sont rassemblées, évaporées et le résidu est repris dans un peu d'éther éthylique. Après filtration du solide on récupère le produit du titre sous forme d'une poudre blanche.
*Point de fusion :* 192-194°C

**Micro-analyse élémentaire :**

| | *C* | *H* | *N* | *S* |
|---|---|---|---|---|
| *% théorique* | 53,44 | 5,16 | 9,35 | 14,27 |
| *% expérimental* | 53,52 | 5,53 | 9,08 | 14,76 |

### ETUDE PHARMACOLOGIQUE DES PRODUITS DE L'INVENTION

### Etude des courants excitateurs induits par l'AMPA dans les oocytes de Xenopus

### a - Méthode :

Des ARNm sont préparés à partir de cortex cérébral de rat mâle Wistar par la méthode au guanidium thiocyanate/phénol/chloroforme. Les ARNm Poly (A⁺) sont isolés par chromatographie sur oligo-dT cellulose et injectés à raison de 50 ng par oocyte. Les oocytes sont laissés 2 à 3 jours en incubation à 18°C pour permettre l'expression des récepteurs puis stockés à 8-10°C.
L'enregistrement électrophysiologique est réalisé dans une chambre en plexiglass® à 20-24°C en milieu OR2 (J. Exp. Zool., 1973, 184, 321-334) par la méthode du «voltage-clamp» à 2 électrodes, une 3^{ème} électrode placée dans le bain servant de référence.

Tous les composés sont appliqués via le milieu d'incubation et le courant électrique est mesuré à la fin de la période d'application. L'AMPA est utilisé à la concentration de 10 µM. Pour chaque composé étudié, on définit la concentration doublant (EC2X) ou quintuplant (EC5X) l'intensité du courant induit par l'AMPA seul (5 à 50 nA).

### b - Résultats :

Les composés de l'invention potentialisent très fortement les effets excitateurs de l'AMPA et leur activité est très nettement supérieure à celle des composés de référence.

A titre d'exemple, le composé de l'Exemple 1 présente une EC2X de 0,1µM.

**COMPOSITION PHARMACEUTIQUE**

| | |
|---|---|
| Formule de préparation pour 1000 comprimés dosés à 100 mg de*N*-(2-{4-[(5,5-dioxido-2,3,3a,4-tétrahydro-1*H*-pyrrolo[2,1-*c*][1,2,4]benzothiadiazin-7-yl)oxy] phényl}éthyl)méthanesulfonamide (exemple 1) | 100 g |
| Hydroxypropycellulose | 2 g |
| Amidon de blé | 10 g |
| Lactose | 100 g |
| Stéarate de magnésium | 3 g |
| Talc | 3 g |

## Revendications

1. Composés de formule (I) : 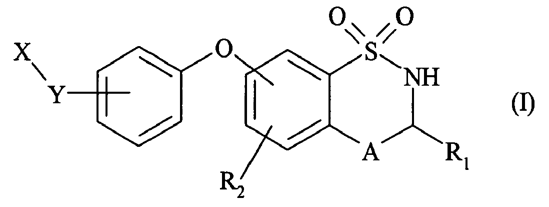 dans laquelle :
R₁ représente un atome d'hydrogène, un groupement alkyle (C₁-C₆) linéaire ou ramifié ou un groupement cycloalkyle (C₃-C₇),
R₂ représente un atome d'hydrogène, d'halogène ou un groupement hydroxy,
A représente un groupement CR₃R₄ ou un groupement NR₃, dans lesquels
R₃ représente un atome d'hydrogène ou un groupement alkyle (C₁-C₆) linéaire ou ramifié,
et R₄ représente un atome d'hydrogène ou d'halogène,
ou bien
A représente un atome d'azote et forme avec le groupement -CHR₁- adjacent le cycle 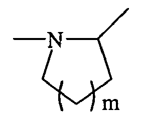 dans lequel m représente 1, 2 ou 3,
Y représente une chaîne alkylène (C₂-C₆) éventuellement substituée par un ou plusieurs groupements identiques ou différents alkyle (C₁-C₆) linéaire ou ramifié ou atomes d'halogène, et dont un des groupements -CH₂- peut être remplacé par un groupement 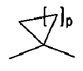 dans lequel p vaut 1, 2, 3 ou 4,
ou Y représente un groupement 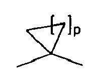 tel que défini précédemment,
X représente un groupement NR₅R₆, S(O)ₙR₇, OR₈, C(O)R₉, amidino (éventuellement substitué par un ou deux groupements identiques ou différents choisis parmi alkyle(C₁-C₆) linéaire ou ramifié, hydroxy, alkoxy (C₁-C₆) linéaire ou ramifié, et 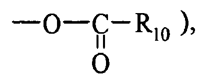 ou un groupement hétérocyclique dans lesquels :
R₅ représente un atome d'hydrogène, un groupement alkyle (C₁-C₆) linéaire ou ramifié, S(O)ₜR₁₁, COR₁₂ ou P(O)OR₁₃OR₁₄,
R₆ représente un atome d'hydrogène, ou un groupement alkyle (C₁-C₆) linéaire ou ramifié,
ou bien R₅ et R₆ forment ensemble avec l'atome d'azote qui les porte un groupement hétérocyclique,
R₈ représente un groupement alkyle (C₁-C₆) linéaire ou ramifié ou un groupement C(O)R₁₅,
R₉ représente un groupement hydroxy, alkoxy (C₁-C₆) linéaire ou ramifié ou amino (éventuellement substitué par un ou deux groupements, identiques ou différents, alkyle (C₁-C₆) linéaire ou ramifié),
R₇, R₁₀, R₁₁, R₁₂, R₁₃, R₁₄ et R₁₅, identiques ou différents, représentent chacun un atome d'hydrogène ; un groupement alkyle (C₁-C₆) linéaire ou ramifié éventuellement substitué par un ou plusieurs atomes d'halogène ; un groupement arylalkyle (C₁-C₆) linéaire ou ramifié ; ou un groupement aryle,
n et t, identiques ou différents, représentent 0, 1 ou 2,
leurs énantiomères et diastéréoisomères ainsi que leurs sels d'addition à un acide ou à une base pharmaceutiquement acceptable.

2. Composés de formule (I), selon la revendication 1, pour lesquels A représente un atome d'azote et forme avec le groupement adjacent ―CHR₁― un cycle 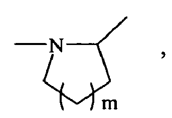 leurs énantiomères et diastéréoisomères ainsi que leurs sels d'addition à un acide ou à une base pharmaceutiquement acceptable.

3. Composés de formule (I), selon la revendication 1, pour lesquels R₂ représente un atome d'hydrogène, leurs énantiomères et diastéréoisomères ainsi que leurs sels d'addition à un acide ou à une base pharmaceutiquement acceptable.

4. Composés de formule (I), selon la revendication 1, pour lesquels Y représente une chaîne alkylène contenant 2 ou 3 atomes de carbone, leurs énantiomères et diastéréoisomères ainsi que leurs sels d'addition à un acide ou à une base pharmaceutiquement acceptable.

5. Composés de formule (I), selon la revendication 1, pour lesquels X représente un groupement NR₅R₆ ou C(O)R₉, leurs énantiomères et diastéréoisomères ainsi que leurs sels d'addition à un acide ou à une base pharmaceutiquement acceptable.

6. Composé de formule (I), selon la revendication 1, qui est le *N*-(2-{4-[(5,5-dioxido-2,3,3a,4-tétrahydro-1*H*-pyrrolo[2,1-*c*][1,2,4]benzothiadiazin-7-yl)oxy]phényl}éthyl) méthanesulfonamide, ses énantiomères et diastéréoisomères ainsi que ses sels d'addition à un acide ou à une base pharmaceutiquement acceptable.

7. Procédé de préparation des composés de formule (I), selon la revendication 1, **caractérisé en ce que** l'on utilise comme produit de départ le composé de formule (II) : 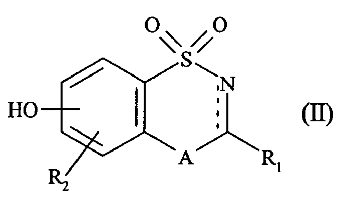 dans laquelle A, R₁ et R₂ sont tels que définis dans la formule (I) et la représentation ----- signifie que la liaison est simple ou double,
que l'on fait réagir, en présence d'acétate de cuivre, avec un dérivé d'acide boronique de formule (III) : 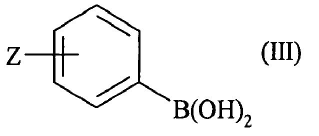 dans laquelle Z représente un groupement acyle (C₁-C₆) linéaire ou ramifié, un groupement -Y-X dans lequel X et Y sont tels que définis dans la formule (I) ou un groupement -Y-X' dans lequel Y est tel que défini dans la formule (I) et X' représente un groupement cyano ou carboxy,
pour conduire directement, ou après réduction (lorsque la représentation ----- représente une double liaison) par un hydrure métallique et/ou par transformation éventuelle du groupement X' ou du groupement acyle, au composé de formule (I) : 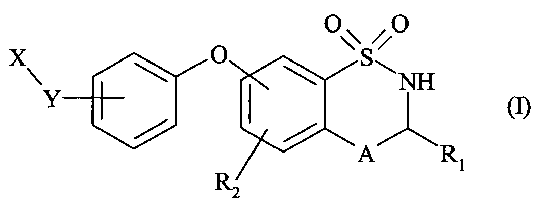 composé de formule (I) que l'on purifie, le cas échéant, selon une technique classique de purification, dont on sépare éventuellement les isomères, selon une technique classique de séparation, et que l'on transforme, si on le souhaite, en ses sels d'addition, à un acide ou à une base pharmaceutiquement acceptable.

8. Compositions pharmaceutiques contenant comme principe actif un composé selon l'une quelconque des revendications 1 à 6 en combinaison avec un ou plusieurs excipients ou véhicules inertes, non toxiques, pharmaceutiquement acceptables.

9. Compositions pharmaceutiques selon la revendication 8 contenant comme principe actif un composé selon l'une quelconque des revendications 1 à 6 utiles en tant que médicaments, comme modulateurs AMPA.
